# FASCICULE DE BREVET EUROPEEN

(11) **EP 3 016 930 B1**
(45) Date de publication et mention de la délivrance du brevet: **09.08.2017**
(21) Numéro de dépôt: 14739892.9
(22) Date de dépôt: 25.06.2014
(51) Int. Cl.: C07C 69/145, C11B 9/00

(54) **EPIMERE (3R)- DE L'ACETATE D'OCTAHYDRO-7,7-DIMETHYL-8-METHYLENE-1H-3A,6-METHANOAZULEN-3-YLE, COMPOSITION, PROCEDE DE SYNTHESE ET UTILISATION DUDIT EPIMERE**
(3R) EPIMER VON OCTAHYDRO-7,7-DIMETHYL-8-METHYLEN-1H-3A,6-METHANOAZULEN-3-YLACETAT, ZUSAMMENSETZUNG, HERSTELLUNGSVERFAHREN UND VERWENDUNG DAVON
(3R) EPIMER OF OCTAHYDRO-7,7-DIMETHYL-8-METHYLEN-1H-3A,6-METHANOAZULEN-3-YL ACETATE, COMPOSITION, PREPARATION PROCESS AND USE THEREOF

(30) Priorité: 03.07.2013 FR 1356482
(43) Date de publication de la demande: 11.05.2016
(73) Titulaire: Robertet S.A., 06130 Grasse (FR); Université de Nice Sophia Antipolis Dirved, 06103 Nice Cedex 2 (FR); Centre National de la Recherche Scientifique (C.N.R.S.), 75794 Paris Cedex 16 (FR)
(72) Inventeur: FILIPPI, Jean-Jacques, F-06100 Nice (FR); BELHASSEN, Emilie, F-06700 Saint-Laurent-du-Var (FR); BALDOVINI, Nicolas, F-06200 Nice (FR); BREVARD, Hugues, F-06130 Grasse (FR)
(74) Mandataire: Santarelli
(86) Numéro de dépôt international: PCT/FR2014/051607
(87) Numéro de publication internationale: WO 2015/001227

(56) Documents cités:
- FR-A1- 2 201 841
- SAKURAI KAZUTOSHI ET AL: "A Simple Preparation of (-)-Khusimone (1) Using Electrochemical Decarboxylative Acetylation", AGRICULTURAL AND BIOLOGICAL CHEMISTRY, JAPAN SOC. FOR BIOSCIENCE, BIOTECHNOLOGY AND AGROCHEM, TOKYO, JP, vol. 53, no. 5, 23 mai 1989 (1989-05-23), pages 1449-1450, XP002719208, ISSN: 0002-1369 cité dans la demande
- JEAN-JACQUES FILIPPI ET AL: "Qualitative and quantitative analysis of vetiver essential oils by comprehensive two-dimensional gas chromatography and comprehensive two-dimensional gas chromatography/mass spectrometry", JOURNAL OF CHROMATOGRAPHY A, vol. 1288, 1 mai 2013 (2013-05-01), pages 127-148, XP055105988, ISSN: 0021-9673, DOI: 10.1016/j.chroma.2013.03.002

## Description

L'invention a pour objet premier l'épimère (*3R*)- de l'acétate d'octahydro-7,7-diméthyl-8-méthylène-1*H*-3a,6-méthanoazulén-3-yle (acétate d'octahydro-7,7-diméthyl-8-méthylène-[3R,3aR,6R,8aR]-1H-3a,6-méthanoazulén-3-yle) de formule I 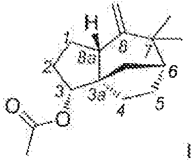 par ailleurs dénommé dans le texte (*R*)-acétate de norzizaényle.

L'invention a également pour objet des compositions comprenant du (*R*)-acétate de norzizaényle ainsi qu'un nouveau procédé de synthèse et l'utilisation dudit ester.

Le terme "Vétiver" désigne en français des plantes de la famille des *Poaceae* (Graminées). Il s'agit de plusieurs espèces du genre *Chrysopogon* (anciennement *Vetiveria*)*.* On en connaît une douzaine d'espèces poussant dans les zones tropicales. La plus connue est *Chrysopogon zizanioides* qui pousse surtout sur le sous-continent indien. Deux autres espèces sont fréquemment cultivées : *Chrysopogon nigritanus* en Afrique australe et *Chrysopogon nemoralis* en Asie du Sud-Est.

La plante se présente sous forme de grandes touffes vertes, dont les racines, se développant verticalement, peuvent atteindre des profondeurs allant jusqu'à trois mètres.

Après distillation, les racines de vétiver fournissent une huile essentielle très visqueuse utilisée en parfumerie. L'huile essentielle de vétiver appartient à la famille olfactive des boisés. C'est une essence à l'odeur fine et complexe: boisée, aromatique, verte, terreuse, quelquefois légèrement fumée ou agrume.

De nombreux parfums sur le marché contiennent de l'huile essentielle de vétiver ou ses dérivés comme ingrédient olfactif clé.

Les études conduites par la demanderesse sur différents extraits, fractions, et dérivés de vétiver ont permis d'identifier plusieurs des principaux composés d'impact olfactif. Parmi ces molécules d'impact, celles présentant un squelette zizaane occupent une place particulière, notamment la khusimone dont la structure correspond à la formule II suivante 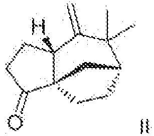 qui est reconnue comme le principe odorant majeur du vétiver. Cette substance présente une odeur boisée, typiquement vétiver et réminiscente de l'odeur de l'essence de vétiver.

L'huile essentielle de vétiver est constituée de multiples composants comme l'ont montré les publications de Weyerstahl (voir par exemple P. Weyerstahl et al., Flavour and Fragrance Journal, 2000, 15, 395-412). On y trouve en particulier des cétones et des alcools. Mais l'essence de vétiver contient naturellement très peu ou pas d'acétate.

Les travaux d'analyse conduits par la demanderesse ont souligné la contribution capitale du (*R*)-acétate de norzizaényle de formule I 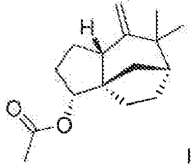 qui est un composé d'impact olfactif majeur et qui présente une note boisée typiquement vétiver très similaire à celle de la khusimone. Lors de ces mêmes travaux, il a été possible de déterminer que son épimère (3S)- présentait un intérêt olfactif bien moindre.

À l'heure actuelle, aucun matériau odorant synthétique correspondant au (R)-acétate de norzizaényle n'est disponible dans le commerce. L'absence de substitut synthétique est en partie due à la nature complexe des sesquiterpènes qui constituent l'essence de vétiver et ses dérivés.

Dans l'art antérieur, on connait la référence CAS : 52771-08-1 dénommée acétate d'octahydro-7,7-diméthyl-8-méthylène-1*H*-3a,6-méthanoazulén-3-yle, sans aucune autre précision quant à la forme isomérique de ce composé (FR 2 201 841).

De même, l'épimère (*3S*)- de l'acétate d'octahydro-7,7-diméthyl-8-méthylène-1*H*-3a,6-méthanoazulén-3-yle (acétate d'octahydro-7,7-diméthyl-8-méthylène-[3*S*,3a*R*,6*R*,8a*R*]-1*H*-3a,6-méthanoazulén-3-yle, par ailleurs dénommé dans le présent texte (*S*)-acétate de norzizaényle) est connu (No. CAS 124601-88-3) (Sakurai Kazutoshi et al., Agricultural and Biological Chemistry, Japan Soc. For Bioscience, Biotechnology and Agrochem, Tokyo, JP, vol. 53, no. 5, 1989-05-23, pages 1449-1450).

Cependant, l'épimère (*3R*) de l'acétate d'octahydro-7,7-diméthyl-8-méthylène-1*H*-3a,6-méthanoazulén-3-yle, [acétate d'octahydro-7,7-diméthyl-8-méthylène-[3*R*,3a*R*,6*R*,8a*R*]-1*H*-3a,6-méthanoazulén-3-yle ou (*R*)-acétate de norzizaényle)] n'a jamais été ni synthétisé ni isolé, particulièrement dans une forme isomérique pure ou quasi pure, c'est-à-dire sous la forme de l'isomère (*R*) à plus de 95%.

La demanderesse après de longues recherches a maintenant mis au point un nouveau procédé de synthèse du (*R*)-acétate de norzizaényle à partir de la khusimone, en 2 étapes.

Ainsi l'invention a pour objet premier l'épimère (3*R*)- de l'acétate d'octahydro-7,7-diméthyl-8-méthylène-1*H*-3a,8-méthanoazulén-3-yle [(acétate d'octahydro-7,7-diméthyl-8-méthylène-[3R,3aR,6R,8aR]-1H-3a,6-méthanoazulén-3-yle)ou (*R*)-acétate de norzizaényle] de formule I

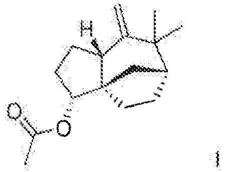

Selon une variante de l'invention ledit composé est isolé.

Par isolé on entend que la solution contenant ledit composé de formule 1 a subi au moins une étape de purification dudit composé.

L'invention a également pour objet une composition comprenant au moins du (R)-acétate de norzizaényle.

Selon une première forme de l'invention la composition peut ne comprendre de l'acétate de norzizaényle que sous sa forme (*R*) [(*R*)-acétate de norzizaényle] et donc ne pas comprendre de forme (*S*)-acétate de norzizaényle et ce quelle que soit la quantité d'acétate de norzizaényle dans la composition.

Selon une autre forme de l'invention, la composition peut comprendre du (*R*)-acétate de norzizaényle et du (*S*)-acétate de norzizaényle dans un rapport en quantité de la forme (*R*)-acétate de norzizaényle sur la forme (*S*)-acétate de norzizaényle [(*R*)/(*S*)] supérieur à 1,2, avantageusement supérieur à 1,5 et ce quelle que soit la quantité totale d'acétate de norzizaényle dans la composition.

L'invention a encore pour objet un nouveau procédé de synthèse du (*R*)-acétate de norzizaényle (Formule I) par réduction de la khusimone (Formule II) en présence d'un solvant organique suivie de l'acétylation de l'alcool obtenu (12-norziza-6(13)-én-2R-ol, Formule III) en présence d'une base, d'un catalyseur nucléophile et d'un solvant aprotique, selon le schéma réactif suivant.

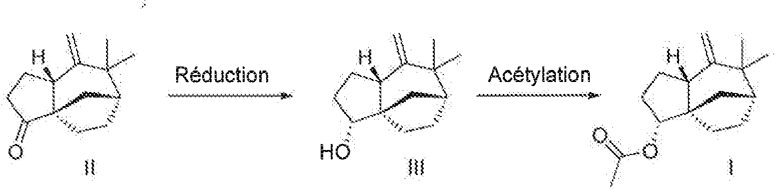

Le nouveau procédé de synthèse proposé ici présente l'avantage, outre sa simplicité, son coût relativement réduit et sa possibilité d'industrialisation, de permettre éventuellement de pouvoir synthétiser le (*R*)-acétate de norzizaényle à l'état pur ou quasi pur. Par pur ou quasi pur, on entend ici qu'en fin de réaction le milieu réactionnel ne contient que très peu, voire pas, d'épimère (*S*) [(*S*)-acétate de norzizaényle)].

En fait l'expérience montre qu'il est possible d'obtenir en deux étapes jusqu'à 95%, voire 98,5%, de la forme (*R*) de l'acétate de norzizaényle dans le milieu réactionnel (voir exemple 1).

L'invention a donc pour objet un procédé de synthèse du (*R*)-acétate de norzizaényle comprenant une première étape de réduction de la khusimone en présence d'un agent réducteur et d'un solvant organique et une seconde étape d'acétylation du produit obtenu à la première étape en présence d'un solvant organique.

Plus précisément la première étape du procédé selon l'invention la réaction de réduction de la khusimone peut être réalisée en présence d'un agent réducteur qui peut être choisi parmi le tétrahydruroaluminate de lithium (LiAlH₄), le DiBAH (Hydrure de düsobutylaluminium), le borohydrure de sodium (NaBH₄), le borohydrure de lithium (LiBH₄), ou le borohydrure de potassium (KBH₄). Préférentiellement, selon l'invention, on pourra utiliser du NaBH₄.

Selon l'invention, la réaction de réduction de la khusimone peut être réalisée avec un rapport molaire [(AR)/(K)] entre l'agent réducteur (AR) et la khusimone (K) qui peut être compris entre 0,5 et 5, préférentiellement, entre 1 et 3, très avantageusement égal à 2.

Selon l'invention la réaction de réduction de la khusimone peut être réalisée en présence d'un solvant organique qui peut être choisi parmi le méthanol (MeOH), l'éthanol (EtOH), le propanol, l'isopropanol, le *n*-butanol, le *sec*-butanol, l'isobutanol le *tert*-butanol, le tétrahydrofurane (THF), le 1,4-dioxane, le diméthylsulfoxyde (DMSO), l'acétonitrile, ou des mélanges en toutes proportions de ces solvants comme par exemple les mélanges DMSO/MeOH, THF/MeOH, DMSO/THF, DMSO/dioxane. Avantageusement, selon l'invention le solvant organique pourra être de l'éthanol.

Selon l'invention, la réduction de la khusimone peut être réalisée à une température qui peut être comprise entre -25°C et la température de reflux du solvant. Avantageusement, la réaction peut être démarrée à une température comprise entre -25°C et 25°C, préférentiellement -25°C et 0°C. La réaction peut ensuite évoluer librement jusqu'à la température de reflux du solvant et ensuite pourra être maintenue à une température comprise entre 25°C et le reflux, jusqu'à consommation des réactifs. Très préférentiellement, une fois atteinte la température de reflux du solvant, la réaction pourra être maintenue à cette température.

L'homme du métier saura sans difficulté arrêter la réaction quand il constatera par prélèvement et analyse que la réaction a atteint l'état d'avancement désiré, par exemple, en mesurant la disparition de la khusimone par analyse en chromatographie gazeuse ou chromatographie sur couche mince, ou encore par résonance magnétique nucléaire.

Selon l'invention, le 12-norziza-6(13)-én-2*R*-ol (formule III) formé à la première étape peut être directement transformé en (*R*)-acétate de norzizaényle (formule I) par acétylation en faisant réagir ledit 12-norziza-6(13)-én-2*R*-ol avec un agent acétylant (Ac) pouvant être choisi parmi l'anhydride acétique, l'acide acétique, ou encore le chlorure d'acétyle, en présence d'une base et d'un catalyseur nucléophile dans un solvant aprotique.

Selon l'invention, la réaction d'acétylation du 12-norziza-6(13)-én-2*R*-ol peut être réalisée avec un rapport molaire [(Ac)/(Z)] entre l'agent acétylant (Ac) et le 12-norziza-6(13)-én-2*R*-ol (Z) qui peut être compris entre 1 et 5, avantageusement égal à 1,5.

Selon l'invention, la réaction d'acétylation du 12-norziza-6(13)-én-2*R*-ol peut être réalisée en présence d'une base organique qui peut être choisie parmi la *N,N-*diisopropyléthylamine (DIPEA), la triéthylamine (Et₃N), ou encore la pyridine. Préférentiellement la réaction d'acétylation du 12-norziza-6(13)-én-2*R*-ol peut être réalisée en présence de triéthylamine.

Selon l'invention, la réaction d'acétylation du 12-norziza-6(13)-én-2*R*-ol peut être réalisée avec un rapport molaire [(BO)/(Z)] entre la base organique (BO) et le 12-norziza-6(13)-én-2*R*-ol (Z) qui peut être compris entre 0,1 et 5, préférentiellement égal à 1,5.

Selon l'invention, la réaction d'acétylation du 12-norziza-6(13)-én-2*R*-ol peut être réalisée en présence d'un catalyseur nucléophile qui peut être choisi parmi la 4-(*N*,*N*-diméthylamino)pyridine (DMAP), le 1,4-diazabicyclo[2.2.2]octane (DABCO), le 1,8-diazabicyclo[5.4.0]undéc-7-ène (DBU), ou encore le *N,N'-*dicyclohexylcarbodiimide (DCC). Préférentiellement selon l'invention on pourra utiliser la DMAP.

Selon l'invention, la réaction d'acétylation du 12-norziza-6(13)-én-2*R*-ol peut être réalisée avec un rapport molaire [(CN)/(Z)] entre le catalyseur nucléophile (CN) et le 12-norziza-6(13)-én-2*R*-ol (Z) qui peut être compris entre 0,01 et 10, préférentiellement égal à 0,1.

Selon l'invention, la réaction d'acétylation du 12-norziza-6(13)-én-2*R*-ol peut être réalisée en présence d'un solvant aprotique qui peut être choisi parmi le *n-*hexane, le cyclohexane, le 1,4-dioxane, le dichlorométhane (DCM), le tétrachlorométhane (CCl₄), le benzène, le trichloroéthylène (Cl₂C=CHCl), le tétrachloroéthylène (Cl₂C=CCl₂), le toluène, le sulfure de carbone (CS₂), le diéthyléther (Et₂O), le chloroforme (CHCl₃), le bromobenzène (PhBr), le chlorobenzène (PhCl), l'acétate d'éthyle (AcOEt), le diméthyléther (DME), le tétrahydrofurane (THF), le 1,1-dichloroéthane (Cl₂CHMe), 1,2-dichloroéthane (ClCH₂CH₂Cl), la pyridine, la butanone, l'acétone, l'anhydride acétique (Ac₂O), la tétraméthylurée ((Me₂N)₂CO), le benzonitrile (PhCN), le propionitrile (CH₃CH₂CN), l'hexaméthylphosphoramide (HMPA), le nitrobenzène (PhNO₂), le nitrométhane (MeNO₂), le diméthylformamide (DMF), l'acétonitrile (MeCN), la sulfolane, le diméthylsulfoxyde (DMSO), le formamide (HCONH₂), le *N*-méthylformamide (NMF), le *N*-méthylacétamide (CH₃CONHMe), l'acide acétique.

Avantageusement selon l'invention le solvant aprotique pourra être du dichlorométhane.

Selon l'invention, la réaction d'acétylation du 12-norziza-6(13)-én-2*R*-ol peut être réalisée à une température qui peut être comprise entre -25°C et la température de reflux du solvant. Avantageusement, la réaction peut être démarrée à une température comprise entre -25°C et 25°C, préférentiellement -25°C et 0°C. La réaction peut ensuite évoluer librement jusqu'à la température de reflux du solvant et ensuite pourra être maintenue à une température comprise autour de 25°C, jusqu'à consommation des réactifs.

L'homme du métier saura sans difficulté arrêter la réaction quand il constatera par prélèvement et analyse que la réaction a atteint l'état d'avancement désiré, par exemple, en mesurant la disparition du 12-norziza-6(13)-én-2*R*-ol par analyse en chromatographie gazeuse ou chromatographie sur couche mince, ou encore par résonance magnétique nucléaire.

L'invention a également pour objet le (*R*)-acétate de norzizaényle de formule I susceptible d'être obtenu par le procédé de synthèse selon l'invention.

L'invention a encore pour objet l'utilisation du (*R*)-acétate de norzizaényle, à titre d'agent parfumant. Avantageusement l'agent parfumant pourra être destiné à entrer dans tout type de compositions comme par exemple un parfum, une eau de parfum, une eau de toilette, des produits d'hygiène, des produits cosmétiques, des savons, des lessives ou encore des bougies.

Selon l'invention le (*R*)-acétate de norzizaényle pourra être utilisé dans des compositions seul, c'est-à-dire sans la présence de la forme (*S*) de l'acétate de norzizaényle ou en présence de la forme (S) de l'acétate de norzizaényle dans un rapport en quantité de la forme (R)-acétate de norzizaényle et la forme (S)-acétate de norzizaényle (R/S) supérieur à 1,2, avantageusement supérieur à 1,5.

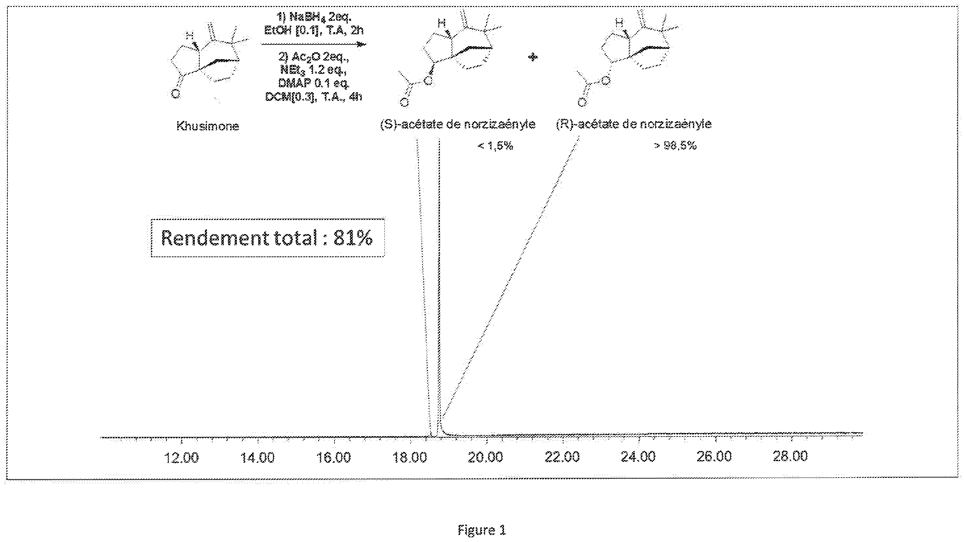

D'autres caractéristiques et avantages de l'invention ressortiront des exemples qui suivent, donnés à titre illustratif et non limitatif ainsi que de la figure 1 qui représente les résultats de l'analyse par chromatographie en phase gazeuse de la réaction de synthèse du (R)-acétate de norzizaényle à partir de la khusimone.

Ainsi la figure 1 présente le chromatogramme obtenu par analyse par chromatographie en phase gazeuse du produit obtenu à l'issu des 2 étapes de la synthèse décrite aux exemples 1 et 2.

### Exemples :

### Exemple 1 : Synthèse du (R)-acétate de norzizaényle à partir de la khusimone :

### Etape 1 : synthèse du 12-norziza-6(13)-én-2R-ol à partir de khusimone

Dans un ballon, 3 mmoles de khusimone sont mises en présence de 6 mmoles de borohydrure de sodium dans 30 mL d'éthanol pour atteindre une concentration en khusimone de 0,1M, à température ambiante pendant 2h. Le solvant est alors évaporé sous vide et le mélange réactionnel est repris dans 10 mL de dichlorométhane. On ajoute ensuite 10 mL d'acide chlorhydrique 1 N. Après décantation, la phase organique est récupérée puis lavée à la saumure, et finalement séchée sur sulfate de magnésium. Après évaporation du solvant, le produit est récupéré sous la forme d'une huile incolore à raison de 97% de rendement.

### Etape 2 : synthèse du (R)-acétate de norzizaényle à partir du 12-norziza-6(13)-én-2R-ol.

Le produit obtenu à l'étape 1 est mis en présence d'anhydride acétique (2 équivalents), de triéthylamine (1,2 équivalents) et de 4-diméthylaminopyridine (0,1 équivalent) dans 10 mL de dichlorométhane, à température ambiante pendant 2h. Le mélange réactionnel est lavé avec une solution d'acide chlorhydrique 0,1 N puis avec une solution saturée de bicarbonate de sodium et enfin avec de la saumure. Après séchage sur sulfate de magnésium et évaporation du solvant le produit est récupéré sous la forme d'une huile incolore qui cristallise spontanément. Le rendement de la réaction s'élève à 83% pour du (R)-acétate de norzizaényle pur à 98,5%.

Le résultat de cette synthèse est présenté à la figure 1 annexée dans laquelle on peut voir le chromatogramme obtenu par analyse par chromatographie en phase gazeuse du produit obtenu à l'issu des 2 étapes de la synthèse schématiquement représentée dans la partie haute de la figure.

On observe la disparition totale du composé de départ (khusimone) et la présence d'un pic majoritaire révélant la présence de (*R*)-acétate de norzizaényle et représentant 98,5% en quantité de la quantité totale d'acétate de norzizaényle (*R*) et (*S*) obtenue. On note également la présence d'un pic minoritaire révélant la présence de (*S*)-acétate de norzizaényle et représentant moins de 1,5% en quantité de la quantité totale d'acétate de norzizaényle (*R*) et (*S*) obtenue.

L'identification des différents composés a été réalisée par spectrométrie de masse, et par résonance magnétique nucléaire après isolement desdits composés.

## Revendications

1. Epimère (*3R*)- de l'acétate d'octahydro-7,7-diméthyl-8-méthylène-1*H*-3a,6-méthanoazulén-3-yle [(acétate d'octahydro-7,7-diméthyl-8-méthylène-[3R,3aR,6R,8aR]-1H-3a,6-méthanoazulén-3-yle) ou (*R*)-acétate de norzizaényle] de formule I

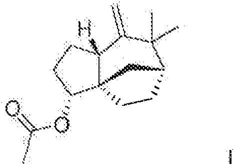

2. (*R*)-acétate de norzizaényle de formule I selon la revendication 1. isolé.

3. Composition comprenant au moins du (*R*)-acétate de norzizaényle de formule I.

4. Composition selon la revendication 3, **caractérisé en ce qu'**elle ne comprend de l'acétate de norzizaényle que sous sa forme (*R*) [100% de (*R*)-acétate de norzizaényle] et ne comprend pas d'acétate de norzizaényle sous sa forme (*S*) [0% de (S)-acétate de norzizaényle].

5. Composition selon la revendication 3, **caractérisé en ce qu'**elle comprend du (*R*)-acétate de norzizaényle et du (*S*)-acétate de norzizaényle dans un rapport en quantité de la forme (*R*)-acétate de norzizaényle sur la forme (*S*)-acétate de norzizaényle [(*R*)/(*S*)] supérieur à 1,2, avantageusement supérieur à 1,5.

6. Procédé de synthèse du (*R*)-acétate de norzizaényle de formule I, comprenant une première étape de réduction de la khusimone en présence d'un agent réducteur et d'un solvant organique et une seconde étape d'acétylation du produit obtenu à la première étape en présence d'un solvant organique.

7. Procédé selon la revendication 6, **caractérisé en ce que** l'agent réducteur est choisi parmi le tétrahydruroaluminate de lithium (LiAlH₄), le DiBAH (Hydrure de diisobutylaluminium), le borohydrure de sodium ((NaBH₄), le borohydrure de lithium (LiBH₄), ou le borohydrure de potassium (KBH₄), préférentiellement le borohydrure de sodium.

8. Procédé selon l'une quelconque des revendications 6 ou 7, **caractérisé en ce que** le rapport molaire [(AR)/(K)] entre l'agent réducteur (AR) et la khusimone (K) est compris entre 0,5 et 5, préférentiellement, entre 1 et 3, très avantageusement égal à 2.

9. Procédé selon l'une quelconque des revendications 6 à 8, **caractérisé en ce que** le solvant organique est choisi parmi le méthanol, l'éthanol, le propanol, l'isopropanol, le *n*-butanol, le *sec*-butanol, l'isobutanol le *tert*-butanol, le tétrahydrofurane (THF), le 1,4-dioxane, le diméthylsulfoxyde (DMSO), l'acétonitrile, ou des mélanges en toutes proportions de ces solvants comme par exemple les mélanges DMSO/méthanol, THF/méthanol, DMSO/THF, DMSO/dioxane, avantageusement l'éthanol.

10. Procédé selon l'une quelconque des revendications 6 à 9, **caractérisé en ce que** la réaction d'acétylation du 12-norziza-6(13)-én-2R-ol est réalisée en faisant réagir ledit 12-norziza-6(13)-én-2R-ol avec un agent acétylant (Ac) en présence d'une base et d'un catalyseur nucléophile dans un solvant aprotique.

11. Procédé selon la revendication 10, **caractérisé en ce que** l'agent acétylant (Ac) est choisi parmi l'anhydride acétique, l'acide acétique, ou encore le chlorure d'acétyle.

12. Procédé selon la revendication 11, **caractérisé en ce que** le rapport molaire [(Ac)/(Z)] entre l'agent acétylant (Ac) et le 12-norziza-6(13)-én-2R-ol (Z) est compris entre 1 et 5, avantageusement égal à 1,5.

13. Procédé selon l'une quelconque des revendications 10 à 12, **caractérisé en ce que** la base organique est choisie parmi la *N*,*N*-diisopropyléthylamine (DIPEA), la triéthylamine (Et₃N), ou encore la pyridine, préférentiellement la triéthylamine.

14. Procédé selon la revendication 13, **caractérisé en ce que** la réaction d'acétylation du 12-norziza-6(13)-én-2R-ol est réalisée avec un rapport molaire BO)/(Z)] entre la base organique (BO) et le 12-norziza-6(13)-én-2R-ol (Z) compris entre 0,1 et 5, préférentiellement égal à 1,5.

15. Procédé selon l'une quelconque des revendications 10 à 14, **caractérisé en ce que** le catalyseur nucléophile est choisi parmi la 4*-*(*N,N-*diméthylamino)pyridine (DMAP), ou encore le 1,4-diazabicyclo[2.2.2]octane (DABCO), le 1,8-diazabicyclo[5.4.0]undéc-7-ène (DBU), le N,N'-dicyclohexylcarbodiimide (DCC), préférentiellement la DMAP.

16. Procédé selon la revendication 15, **caractérisé en ce que** la réaction d'acétylation du 12-norziza-6(13)-én-2R-ol est réalisée avec un rapport molaire [(CN)/(Z)] entre le catalyseur nucléophile (CN) et le 12-norzizaénol (Z) compris entre 0,01 et 10, préférentiellement égal à 0,1.

17. Procédé selon l'une quelconque des revendications 10 à 16, **caractérisé en ce que** le solvant aprotique est choisi parmi le *n*-hexane, le cyclohexane, le 1,4-dioxane, le dichlorométhane (DCM), le tétrachlorométhane (CCl₄), le benzène, le trichloroéthylène (Cl₂C=CHCl), le tétrachloroéthylène (Cl₂C=CCl₂), le toluène, le sulfure de carbone (CS₂), le diéthyléther (Et₂O), le chloroforme (CHCl₃), le bromobenzène (PhBr), le chlorobenzène (PhCl), l'acétate d'éthyle (AcOEt), le diméthyléther (DME), le tétrahydrofurane (THF), le 1,1-dichloroéthane (Cl₂CHMe), 1,2-dichloroéthane (ClCH₂CH₂Cl), la pyridine, la butanone, l'acétone, l'anhydride acétique (Ac₂O), la tétraméthylurée ((Me₂N)₂CO), le benzonitrile (PhCN), le propionitrile (CH₃CH₂CN), l'hexaméthylphosphoramide (HMPA), le nitrobenzène (PhNO₂), le nitrométhane (MeNO₂), le diméthylformamide (DMF), l'acétonitrile (MeCN), la sulfolane, le diméthylsulfoxyde (DMSO), le formamide (HCONH₂), le *N*-méthylformamide (NMF), le *N*-méthylacétamide (CH₃CONHMe), l'acide acétique, avantageusement le dichlorométhane.

18. (*R*)-acétate de norzizaényle de formule I susceptible d'être obtenu par le procédé de synthèse selon l'invention.

19. Utilisation du (*R*)-acétate de norzizaényle à titre d'agent parfumant.

20. Utilisation selon la revendication 19, **caractérisé en ce que** le (*R*)-acétate de norzizaényle est seul sans la présence de la forme (*S*) de l'acétate de norzizaényle ou en présence de la forme (*S*) de l'acétate de norzizaényle dans un rapport en quantité de la forme (*R*)-acétate de norzizaényle et la forme (S)-acétate de norzizaényle (R/S) supérieur à 1,2, avantageusement supérieur à 1,5.

## Patentansprüche

1. (3*R*)-Epimer von Octahydro-7,7-dimethyl-8-methylen-1*H*-3a, 6-methanoazulen-3-yl-acetat [(Octahydro-7,7-dimethyl-8-methylen-[3R, 3aR,6R,8aR]-1 H-3a,6-methanoazulen-3-yl-acetat) oder (*R*)-Acetat von Norzizaenyl] von Formel I

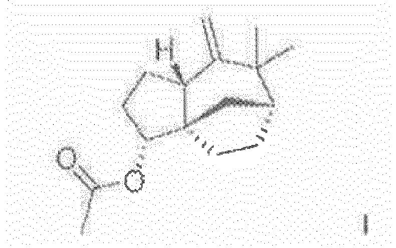

2. (*R*)-Acetat von Norzizaenyl von Formel I nach Anspruch 1, isoliert.

3. Zusammensetzung, die wenigstens (*R*)-Acetat von Norzizaenyl von Formel I umfasst.

4. Zusammensetzung nach Anspruch 3, **dadurch gekennzeichnet, dass** sie Norzizaenylacetat nur in seiner (*R*)-Form [100% (R)-Acetat von Norzizaenyl] umfasst und kein Norzizaenylacetat in seiner (*S*)-Form [0% (S)-Acetat von Norzizaenyl] umfasst.

5. Zusammensetzung nach Anspruch 3, **dadurch gekennzeichnet, dass** sie (R)-Acetat von Norzizaenyl und (*S*)-Acetat von Norzizaenyl in einem Mengenverhältnis der Form (R)-Acetat von Norzizaenyl zur Form (S)-Acetat von Norzizaenyl [(*R*)/(*S*)] von größer als 1,2, vorteilhafterweise größer als 1,5 enthält.

6. Verfahren zum Synthetisieren von (*R*)-Acetat von Norzizaenyl von Formel I, das einen ersten Schritt, die Reduktion von Khusimon in Anwesenheit eines Reduktionsmittels und eines organischen Lösungsmittels, und einen zweiten Schritt beinhaltet, die Acetylierung des im ersten Schritt erhaltenen Produkts in Anwesenheit eines organischen Lösungsmittels.

7. Verfahren nach Anspruch 6, **dadurch gekennzeichnet, dass** das Reduktionsmittel ausgewählt wird aus Lithiumaluminiumhydrid (LiAlH₄), DiBAH (Diisobutylaluminiumhydrid), Natriumborhydrid (NaBH₄), Lithiumborhydrid (LiBH₄) oder Kaliumborhydrid (KBH₄), vorzugsweise Natriumborhydrid.

8. Verfahren nach Anspruch 6 oder 7, **dadurch gekennzeichnet, dass** das Molverhältnis [(RM)/(K)] zwischen dem Reduktionsmittel (RM) und dem Khusimon (K) zwischen 0,5 und 5, vorzugsweise zwischen 1 und 3, sehr vorteilhafterweise bei gleich 2 liegt.

9. Verfahren nach einem der Ansprüche 6 bis 8, **dadurch gekennzeichnet, dass** das organische Lösungsmittel ausgewählt wird aus Methanol, Ethanol, Propanol, Isopropanol, *n*-Butanol, *sec*-Butanol, Isobutanol, *tert*-Butanol, Tetrahydrofuran (THF), 1,4-Dioxan, Dimethylsulfoxid (DMSO), Acetonitril und Mischungen in allen Proportionen dieser Lösungsmittel, wie zum Beispiel die Mischungen DMSO/Methanol, THF/Methanol, DMSO/THF, DMSO/Dioxan, vorteilhafterweise Ethanol.

10. Verfahren nach einem der Ansprüche 6 bis 9, **dadurch gekennzeichnet, dass** die Acetylierungsreaktion von 12-Norziza-6(13)-en-2R-ol durch Umsetzen vom genannten 12-Norziza-6(13)-en-2R-ol mit einem Acetylierungsmittel (Ac) in Anwesenheit einer Base und eines nukleophilen Katalysators in einem aprotischen Lösungsmittel durchgeführt wird.

11. Verfahren nach Anspruch 10, **dadurch gekennzeichnet, dass** das Acetylierungsmittel (Ac) ausgewählt wird aus Essigsäureanhydrid, Essigsäure oder Acetylchlorid.

12. Verfahren nach Anspruch 11, **dadurch gekennzeichnet, dass** das Molverhältnis [(Ac)/(Z)] zwischen dem Acetylierungsmittel (Ac) und 12-Norziza-6(13)-en-2R-ol (Z) zwischen 1 und 5, vorteilhafterweise bei gleich 1,5 liegt.

13. Verfahren nach einem der Ansprüche 10 bis 12, **dadurch gekennzeichnet, dass** die organische Base ausgewählt wird aus *N*,*N*-Diisopropylethylamin (DIPEA), Triethylamin (Et₃N) oder Pyridin, vorzugsweise Triethylamin.

14. Verfahren nach Anspruch 13, **dadurch gekennzeichnet, dass** die Acetylierungsreaktion von 12-Norziza-6(13)-en-2R-ol mit einem Molverhältnis [(OB)/(Z)] zwischen der organischen Base (OB) und 12-Norziza-6(13)-en-2R-ol (Z) zwischen 0,1 und 5, vorzugsweise von gleich 1,5 durchgeführt wird.

15. Verfahren nach einem der Ansprüche 10 bis 14, **dadurch gekennzeichnet, dass** der nukleophile Katalysator ausgewählt wird aus 4-(*N*,*N*-Dimethylamino)pyridin (DMAP) und 1,4-Diazabicyclo[2.2.2]octan (DABCO), 1,8-Diazabicyclo[5.4.0]undec-7-en (DBU) und N,N'-Dicyclohexylcarbodiimid (DCC), vorzugsweise DMAP.

16. Verfahren nach Anspruch 15, **dadurch gekennzeichnet, dass** die Acetylierungsreaktion von 12-Norziza-6(13)-en-2R-ol mit einem Molverhältnis [(NK)/(Z)] zwischen dem nukleophilen Katalysator (NK) und 12-Norzizaenol (Z) zwischen 0,01 und 10, vorzugsweise von gleich 0,1 durchgeführt wird.

17. Verfahren nach einem der Ansprüche 10 bis 16, **dadurch gekennzeichnet, dass** das aprotische Lösungsmittel ausgewählt wird aus *n*-Hexan, Cyclohexan, 1,4-Dioxan, Dichlormethan (DCM), Tetrachlormethan (CCl₄), Benzol, Trichlorethylen (Cl₂C=CHCl), Tetrachlorethylen (Cl₂C=CCl₂), Toluol, Kohlenstoffdisulfid (CS₂), Diethylether (Et₂O), Chloroform (CHCl₃), Brombenzol (PhBr), Chlorbenzol (PhCl), Ethylacetat (AcOEt), Dimethylether (DME), Tetrahydrofuran (THF), 1,1-Dichlorethan (Cl₂CHMe), 1,2-Dichlorethan (ClCH₂CH₂Cl), Pyridin, Butanon, Aceton, Essigsäureanhydrid (Ac₂O), Tetramethylharnstoff ((Me₂N)₂CO), Benzonitril (PhCN), Propionitril (CH₃CH₂CN), Hexamethylphosphoramid (HMPA), Nitrobenzol (PhNO₂), Nitromethan (MeNO₂), Dimethylformamid (DMF), Acetonitril (MeCN), Sulfolan, Dimethylsulfoxid (DMSO), Formamid (HCONH₂), *N*-Methylformamid (NMF), *N*-Methylacetamid (CH₃CONHMe), Essigsäure, vorteilhafterweise Dichlormethan.

18. (*R*)-Acetat von Norzizaenyl von Formel I, das mit dem Syntheseverfahren gemäß der Erfindung erhalten werden kann.

19. Verwendung von (*R*)-Acetat von Norzizaenyl als Parfümiermittel.

20. Verwendung nach Anspruch 19, **dadurch gekennzeichnet, dass** das (*R*)-Acetat von Norzizaenyl allein ohne Anwesenheit der (*S*)-Form des Acetats von Norzizaenyl oder in Anwesenheit der (*S*)-Form des Acetats von Norzizaenyl in einem Mengenverhältnis der Form (*R*)-Acetat von Norzizaenyl und der Form (*S*)-Acetat von Norzizaenyl (R/S) von größer als 1,2, vorteilhafterweise größer als 1,5 vorliegt.

## Claims

1. (3*R*)-epimer of octahydro-7,7-dimethyl-8-methylene-1*H*-3a,6-methanoazulen-3-yl acetate [(octahydro-7,7-dimethyl-8-methylene-[3R,3aR,6R,8aR]-1H-3a,6-methanoazulen-3-yl acetate) or (*R*)-norzizaenyl acetate] of formula I

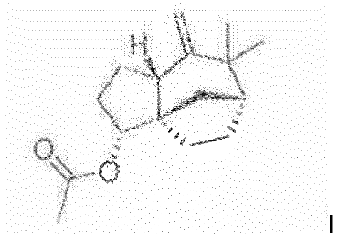

2. (*R*)-norzizaenyl acetate of formula I according to claim 1, isolated.

3. Composition comprising at least (*R*)-norzizaenyl acetate of formula I.

4. Composition according to claim 3, **characterized in that** it comprises norzizaenyl acetate in its (*R*) form only [100% (*R*)-norzizaenyl acetate] and does not comprise norzizaenyl acetate in its (*S*) form [0% (S)-norzizaenyl acetate].

5. Composition according to claim 3, **characterized in that** it comprises (*R*)-norzizaenyl acetate and (*S*)-norzizaenyl acetate in a quantity ratio of the (*R*)-norzizaenyl acetate form to the (*S*)-norzizaenyl acetate form [(*R*)/(*S*)] greater than 1.2, advantageously greater than 1.5.

6. Method for the synthesis of (*R*)-norzizaenyl acetate of formula I, comprising a first step of reduction of khusimone in the presence of a reducing agent and an organic solvent, and a second step of acetylation of the product obtained in the first step in the presence of an organic solvent.

7. Method according to claim 6, **characterized in that** the reducing agent is selected from lithium aluminium hydride (LiAlH₄), DiBAH (diisobutylaluminium hydride), sodium borohydride (NaBH₄), lithium borohydride (LiBH₄) or potassium borohydride (KBH₄), preferably sodium borohydride.

8. Method according to any one of claims 6 or 7, **characterized in that** the molar ratio [(RA)/(K)] between the reducing agent (RA) and the khusimone (K) is comprised between 0.5 and 5, preferably between 1 and 3, very advantageously equal to 2.

9. Method according to any one of claims 6 to 8, **characterized in that** the organic solvent is selected from methanol, ethanol, propanol, isopropanol, *n*-butanol, *sec*-butanol, isobutanol, *tert*-butanol, tetrahydrofuran (THF), 1,4-dioxane, dimethylsulphoxide (DMSO), acetonitrile, or mixtures in all proportions of these solvents, such as for example DMSO/methanol, THF/methanol, DMSO/THF, DMSO/dioxane mixtures, advantageously ethanol.

10. Method according to any one of claims 6 to 9, **characterized in that** the acetylation reaction of 12-nor-ziza-6(13)-en-2R-ol is carried out by reacting said 12-nor-ziza-6(13)-en-2R-ol with an acetylating agent (Ac) in the presence of a base and a nucleophilic catalyst in an aprotic solvent.

11. Method according to claim 10, **characterized in that** the acetylating agent (Ac) is selected from acetic anhydride, acetic acid, or also acetyl chloride.

12. Method according to claim 11, **characterized in that** the molar ratio [(Ac)/(Z)] between the acetylating agent (Ac) and 12-nor-ziza-6(13)-en-2R-ol (Z) is comprised between 1 and 5, advantageously equal to 1.5.

13. Method according to any one of claims 10 to 12, **characterized in that** the organic base is selected from *N*,*N*-diisopropylethylamine (DIPEA), triethylamine (Et₃N) or also pyridine, preferably triethylamine.

14. Method according to claim 13, **characterized in that** the acetylation reaction of 12-nor-ziza-6(13)-en-2R-ol is carried out with a molar ratio [(OB)/(Z)] between the organic base (OB) and the 12-nor-ziza-6(13)-en-2R-ol (Z) comprised between 0.1 and 5, preferably equal to 1.5.

15. Method according to any one of claims 10 to 14, **characterized in that** the nucleophilic catalyst is selected from 4-(*N*,*N*-dimethylamino)pyridine (DMAP), or also 1,4-diazabicyclo[2.2.2]octane (DABCO), 1,8-diazabicyclo[5.4.0]undec-7-ene (DBU) or N,N' -dicyclohexylcarbodiimide (DCC), preferably DMAP.

16. Method according to claim 15, **characterized in that** the acetylation reaction of 12-nor-ziza-6(13)-en-2R-ol is carried out with a molar ratio [(NC)/(Z)] between the nucleophilic catalyst (NC) and the 12-nor-zizaenol (Z) comprised between 0.01 and 10, preferably equal to 0.1.

17. Method according to any one of claims 10 to 16, **characterized in that** the aprotic solvent is selected from *n*-hexane, cyclohexane, 1,4-dioxane, dichloromethane (DCM), carbon tetrachloride (CCl₄), benzene, trichloroethylene (Cl₂C=CHCl), tetrachloroethylene (Cl₂C=CCl₂), toluene, carbon disulphide (CS₂), diethyl ether (Et₂O), chloroform (CHCl₃), bromobenzene (PhBr), chlorobenzene (PhCl), ethyl acetate (AcOEt), dimethyl ether (DME), tetrahydrofuran (THF), 1,1-dichloroethane (Cl₂CHMe), 1,2-dichloroethane (ClCH₂CH₂Cl), pyridine, butanone, acetone, acetic anhydride (Ac₂O), tetramethylurea ((Me₂N)₂CO), benzonitrile (PhCN), propionitrile (CH₃CH₂CN), hexamethylphosphoramide (HMPA), nitrobenzene (PhNO₂), nitromethane (MeNO₂), dimethylformamide (DMF), acetonitrile (MeCN), sulpholane, dimethylsulphoxide (DMSO), formamide (HCONH₂), *N-*methylformamide (NMF), *N*-methylacetamide (CH₃CONHMe), acetic acid, advantageously dichloromethane.

18. (*R*)-norzizaenyl acetate of formula I capable of being obtained by the synthesis method according to the invention.

19. Use of (*R*)-norzizaenyl acetate as a fragrance agent.

20. Use according to claim 19, **characterized in that** the (*R*)-norzizaenyl acetate is alone, without the presence of the (*S)*-norzizaenyl acetate form, or in the presence of the (*S*)-norzizaenyl acetate form, in a quantity ratio of the (*R*)-norzizaenyl acetate form and the (S)-norzizaenyl acetate form (R/S) greater than 1.2, advantageously greater than 1.5.
